# EUROPEAN PATENT APPLICATION

(11) **EP 2 578 698 A1**
(43) Date of publication of application: **10.04.2013**
(21) Application number: 11184398.3
(22) Date of filing: 07.10.2011
(51) Int. Cl.: C12Q 1/68

(54) **Genetic polymorphisms associated with venous thrombosis in women, methods of detection and uses thereof**

(71) Applicant: Scharf, Rüdiger Eberhard, 40593 Düsseldorf (DE); Zotz, Rainer Bernd, 40210 Düsseldorf (DE); Gerhardt, Andrea, 89073 Ulm (DE); Szafarczyk, Daniel, 40699 Düsseldorf (DE)
(72) Inventor: Scharf, Rüdiger Eberhard, 40593 Düsseldorf (DE); Zotz, Rainer Bernd, 40210 Düsseldorf (DE); Gerhardt, Andrea, 89073 Ulm (DE); Szafarczyk, Daniel, 40699 Düsseldorf (DE)
(74) Representative: Kröncke, Rolf

(57) **Abstract**

Methods and kits for identifying a woman having an increased risk of developing venous thrombosis, requiring prophylactic or therapeutic treatment are provided, and methods for identifying an agent useful in prophylactically or therapeutically treating venous thrombosis in women, said methods comprising genotyping the woman with respect to the F9 G32023A (rs440051) polymorphism and/or the F9 5:32164:6/4 polymorphism (rs35599944).

SEQUENCE LISTING

<110>  HEINRICH-HEINE-UNIVERSITAET DUESSELDORF

<120>  Genetic polymorphisms associated with venous thrombosis in women,
        methods of detection and uses thereof

<130>  UD 40317

<160>  8

<170>  PatentIn version 3.5

<210>  1
<211>  52
<212>  DNA
<213>  Homo sapiens

<220>
<221>  SNP
<222>  (27)..(27)
<223>  Public SNP ID: rs440051
       Polymorphism comprises an R residue at position 27 within the
       nucleotide sequence. For F9 A32023A said R is an A.

<220>
<221>  SNP
<222>  (27)..(27)
<223>  Public SNP ID: rs440051
       Polymorphism consists of an R residue at position 27 within the
       nucleotide sequence. For F9 A32023A said R is an A.

<400>  1
tgtctatcaa acccagactt gcttccatag tggagacttg cttttcagaa ca
52

<210>  2
<211>  52
<212>  DNA
<213>  Homo sapiens

<220>
<221>  SNP
<222>  (27)..(27)
<223>  Public SNP ID: rs440051
       Polymorphism consists of an R residue at position 27 within the
       nucleotide sequence. For F9 G32023A said R is a G.

<400>  2
tgtctatcaa acccagactt gcttccgtag tggagacttg cttttcagaa ca
52

<210>  3
<211>  59
<212>  DNA
<213>  Homo sapiens

<220>
<221>  SNP
<222>  (27)..(34)
<223>  Public SNP ID: rs35599944
       Polymorphism consists of 4 to 6 GT-dinucleotides. For F9
       4:32164:6/4 the polymorphism conssists of 4 GT-dinucleotides,
       beginning at position 27 of the nucleotide sequeence.

<400>  3
tatataatat acaatataaa tatatagtgt gtgtatgcgt gtgtgtagac acacacgca
59



<210>  4
<211>  61
<212>  DNA
<213>  Homo sapiens



<220>
<221>  SNP
<222>  (27)..(36)
<223>  Public SNP ID: rs35599944
       Polymorphism consists of 4 to 6 GT-dinucleotides. For F9
       5:32164:6/4 the polymorphism consists of 5 GT-dinucleotides,
       beginning at position 27 of the nucleotide sequence.

<400>  4
tatataatat acaatataaa tatatagtgt gtgtgtatgc gtgtgtgtag acacacacgc
60

a
61



<210>  5
<211>  63
<212>  DNA
<213>  Homo sapiens



<220>
<221>  SNP
<222>  (27)..(38)
<223>  Public SNP ID: rs35599944
       Polymorphism consists of 4 to 6 GT-dinucleotides. For F9
       6:32164:6/4 the polymorphism consists of 6 GT-dinucleotides,
       beginning at position 27 of the nucleotide sequence.

<400>  5
tatataatat acaatataaa tatatagtgt gtgtgtgtat gcgtgtgtgt agacacacac
60

gca
63



<210>  6

<211>  801
<212>  DNA
<213>  Homo sapiens

<220>
<221>  SNP
<222>  (401)..(401)
<223>  Public SNP ID: rs440051

<220>
<221>  SNP
<222>  (556)..(563)
<223>  Public SNP ID: rs35599944
F9 4:32164:6/4

<400>  6
tctccactat ggcaactaac tcactcaatt ttccctcctt agcagcattc catcttcccg
60

atcttctttg cttctccaac caaaacatca atgtttatta gttctgtata cagtacagga
120

tctttggtct actctatcac aaggccagta ccacactcat gaagaaagaa cacaggagta
180

gctgagaggc taaaactcat caaaaacact actccttttc ctctacccta ttcctcaatc
240

ttttaccttt tccaaatccc aatccccaaa tcagtttttc tctttcttac tccctctctc
300

ccttttaccc tccatggtcg ttaaaggaga gatggggagc atcattctgt tatacttctg
360

tacacagtta tacatgtcta tcaaacccag acttgcttcc rtagtggaga cttgcttttc
420

agaacatagg gatgaagtaa ggtgcctgaa aagtttgggg gaaaagtttc tttcagagag
480

ttaagttatt ttatatatat aatatatata taaaatatat aatatacaat ataaatatat
540

agtgtgtgtg tatgcgtgtg tgtagacaca cacgcataca cacatataat ggaagcaata
600

agccattcta agagcttgta tggttatgga ggtctgacta ggcatgattt cacgaaggca
660

agattggcat atcattgtaa ctaaaaaagc tgacattgac ccagacatat tgtactcttt
720

ctaaaaataa taataataat gctaacagaa agaagagaac cgttcgtttg caatctacag
780

ctagtagaga ctttgaggaa g
801

<210>  7
<211>  19
<212>  DNA
<213>  Artificial Sequence

<220>
<223>  sense primer for amplification of a fragment of the Homo sapiens
       F9 gene

<400>  7
ccctccatgg tcgttaaag
19



<210>  8
<211>  24
<212>  DNA
<213>  Artificial Sequence

<220>
<223>  5' primer for amplification of a fragment of the Homo sapiens F9
       gene.

<400>  8
gtacaatatg tctgggtcaa tgtc
24

## Description

The present invention relates to the field of diagnosing and treating thrombosis. More specifically, the present invention relates to specific polymorphisms in the genome of women, and to the association of these polymorphisms with venous thrombosis in women. Based on the allele frequencies described herein, the naturally occurring polymorphisms that are disclosed herein are associated with venous thrombosis in women, and can be used for assessing a woman's risk of developing venous thrombosis, for early detection of venous thrombosis in a woman, for providing clinically relevant information for preventing venous thrombosis, for treating venous thrombosis, for providing a kit for assessing a woman's risk of developing venous thrombosis, and as target for designing diagnostic reagents. In addition, said polymorphisms may be used for screening and assessing the efficacy of therapeutic agents. Methods, assays, kits and reagent for detecting the presence of these polymorphisms are provided.

Thrombosis is the formation of a blood clot inside a blood vessel, wherein said clot obstructs the flow of blood through said blood vessel. If the thrombus occupies more than 75% of the diameter of an artery, the blood flow through this artery is reduced that much causing clinical symptoms due to the decreased oxygen supply of the tissue that should be supplied. In addition, metabolic products such as lactic acid accumulate. An obstruction of more than 90% of an artery's diameter will result in anoxia, infarction and tissue necrosis. When a clot breaks free, the traveling clot is called an embolus. The embolus may lodge in and obstruct a blood vessel somewhere behind the initial occlusion.

Venous thrombosis denotes the formation of a blood clot in a vein. Several diseases may be classified as venous thrombosis: deep vein thrombosis, portal vein thrombosis, renal vein thrombosis, jugular vein thrombosis, Budd-Chiari-Syndrome, Paget-von-Schroetter syndrome, and cerebral sinus venous thrombosis. A venous thrombosis is a chronic and polygenic disease. Approximately 1‰ of US citizens encounter a venous thrombotic event for the first time each year resulting in over 200,000 new cases annually. Roughly, 30% of these patients die within three days.

Venous thrombosis is a multi-factorial disease resulting from acquired factors and genetic factors. For the latter, there is a need for genetic markers that are predictive of a person's predisposition to venous thrombosis such that humans having an increased risk of developing venous thrombosis can be identified and prophylactically treated. For example, WO 2008/051511 A2 discloses genetic polymorphisms that are associated with venous thrombosis, and describes methods of detecting these polymorphisms, and of using these polymorphisms. The claimed method comprises detecting a single nucleotide polymorphism as specified in any one of 146 specific nucleotide sequences or complements thereof, wherein the presence of the single nucleotide polymorphism is indicative of an altered risk for venous thrombosis. WO 2008/051511 A2 is incorporated herein in its entirety by virtue of reference and/or by virtue of recitation.

Venous thromboses occur more often in elderly patients and constitute an important problem in health care policies. The pathomechanism of an increased likelihood of encountering a venous thrombosis event at later ages is insufficiently discovered. Therefore, there is a need for additional and/or alternative genetic markers that are indicative for the predisposition of an elderly person to develop venous thrombosis. It is known that the activity of Factor IX increases with a person's age, and Kurachi *et al.* (Kurachi s, Deyashiki Y, Takeshita J, Kurachi K. Genetic Mechanisms of Age Regulation of Human Blood coagulation Factor IX. Science, 1999, 285: 739-743; Kurachi K, Kurachi. Molecular mechanisms of age-related regulation of genes. J Thromb Haemost. 2005, 3: 909-914) identified an age-related increase element in close proximity to the Factor IX gene in rodents which modulates the age-related increase in Factor IX activity. It was also shown that an increased activity of factor IX is associated with an increase of the risk of developing venous thrombosis.

Factor IX is a serine protease of the peptidase S1 family. Factor IX is synthesized as an inactive precursor protein that is activated in that its signal peptide is removed, and the resulting preprotein is subsequently cleaved into two subunits which are linked to one another by a disulfide bond. The cleavage into its two subunits is performed either by factor XIa or by factor VIIa. The cleavage into its two subunits activates factor IX into factor IXa. Factor IXa in turn hydrolyses factor X of the coagulation cascade into factor Xa in the presence of Ca²⁺, membrane lipids and cofactor VIII.

The factor IX-gene, also designated as factor 9-gene, contains the genetic code for clotting factor IX. The factor IX-gene is located on the long arm of the X-chromosome, and has a length of approximately 32,000 base pairs. The genetic code for clotting factor IX is distributed to 8 exons. Its 7 introns account for 92% of the length of the factor IX-gene. The single nucleotide polymorphism database (dbSNP) of the National Center for Biotechnology Information (NCBI) describes approximately 200 polymorphisms within the factor IX gene. However, only one of those polymorphisms was associated with an alteration of the risk of developing thrombosis. Bezemer *et al.* (Bezemer ID, Bare LA, Doggen CJ, Arellano AR, Tong C, Rowland CM, Catanese J, Young BA, Reitsma, PH, Devlin JJ, Rosendaal FR. Gene variants associated with deep vein thrombosis. JAMA. 2008, 299: 1306-1314) have shown a direct correlation of the polymorphism Factor IX - Malmö (rs6048) within exon 6 of the factor IX gene and the risk of developing thrombosis. The less frequent allele "G" (mutation) occurred at a frequency of 32% (minor allele frequency = 0.32), and was associated with a 15 to 43 % reduction of thrombosis risk compared to the "wild-type" allele "A". The mechanism remained enigmatic, in particular because no alteration of factor IX activity was observed.

Another rare mutation of factor IX gene, factor IX - Padua, is supposed to be associated with a person's risk of developing a thrombosis. Simioni *et al.* (Simioni P, Tormene D, Tognin G, Gavasso S, Bulato C, lacobelli NP, Finn JD, Spiezia L, Radu C, Arruda VR. X-linked thrombophilia with mutant factor IX (factor IX Padua). N Engl J Med. 2009, 361: 1671-1675) identified a mutation in exon 8 of the factor IX-gene, the wild type allele "G" was replaced with allele "T", in a patient suffering from thrombosis. That patient exhibited increased factor IX activity, and it was also shown *in-vitro* that factor IX - Padua is associated with an increased activity of factor IX activity. However, factor IX - Padua is a rare mutation.

Based on afore-mentioned information, coagulation factor IX was investigated for identifying genetic variations that are associated with age-related effects, and to identify genetic variants which constitute a risk factor with respect to thrombosis in elderly persons. During said investigation, it was surprisingly found that two genetic variants of the factor 9 (F9) gene, namely genotype AA of the F9 G32023A polymorphism (rs440051) and genotype GT6/GT6 of the F9 5:32164:6/4 polymorphism (rs35599944), which are correlated with one another, are associated with an eleven-fold increase of the risk of women to encounter a thrombolic event. This finding appears contradictory to the results of van Minkelen et al. (van Minkelen R, de Visser MCH, van Hylckama Vlieg A, Vos, HL, Bertina RM Sequence variants and haplotypes of the factor IX gene and the risk of venous thrombosis. J Thromb Haemostat 2008, 6: 1610-1613) who reported that the first of said two polymorphisms does not have an effect on deep venous thrombosis in women.

The first polymorphism refers to registration number rs440051 within the single nucleotide polymorphism database of the NCBI. The first polymorphism is designated F9 G32023A for specific non-limiting embodiments of the present invention. The number 32023 within this designation identifies the position of the polymorphism within the Homo sapiens X-chromosome, beginning with the first nucleotide of the Factor IX (F9) gene, i.e. number 1 represents the first nucleotide of the first exon of the Factor IX-gene, with respect to reference sequence NC_000023.9 (gi:89161218, Homo sapiens chromosome X, reference assembly, complete sequence: NCBI) of the X-chromosome. The Factor IX-gene is positioned from nucleotide 138440561 to nucleotide 138473283 within said X-chromosome reference sequence NC_000023.9.

The other polymorphism refers to registration number rs35599944 within the single nucleotide polymorphism database of the NCBI. This polymorphism is designated F9 5:32164:6/4 for specific non-limiting embodiments of the present invention. The number 32164 within this designation identifies the position of the polymorphism within the Homo sapiens X-chromosome, beginning with the first nucleotide of the Factor IX (F9) gene, i.e. number 1 represents the first nucleotide of the first exon of the Factor IX-gene, with respect to reference sequence NC_000023.9 (gi:89161218, Homo sapiens chromosome X, reference assembly, complete sequence: NCBI) of the X-chromosome. Hence, polymorphism rs35599944 begins 141 bp downstream of polymorphism rs440051 as can be inferred from SEQ ID No: 6 which represents a fragment of the Homo sapiens Factor IX-gene including polymorphisms rs440051 and rs35599944.

In a first aspect, the invention concerns a method for identifying a woman who has an increased risk of developing venous thrombosis, the method comprises detecting the presence of genotype AA of the F9 G32023A polymorphism, and/or the presence of the genotype GT6/GT6 of the F9 5:32164:6/4 polymorphism, wherein the presence of either of said genotypes or the presence of both of said genotypes is indicative for the increased risk for venous thrombosis in said woman.

In a second aspect, the invention concerns a method for identifying a woman who is in need of receiving therapeutically or prophylactic treatment of venous thrombosis, comprising detecting the presence of genotype AA of the F9 G32023A polymorphism, and/or the presence of the genotype GT6/GT6 of the F9 5:32164:6/4 polymorphism.

In a third aspect, the invention concerns a method for prophylactically or therapeutically treating a woman who is in need of receiving prophylactic or therapeutic treatment of venous thrombosis, comprising detecting the presence of genotype AA of the F9 G32023A polymorphism, and/or the presence of the genotype GT6/GT6 of the F9 5:32164:6/4 polymorphism, and treating said woman prophylactically or therapeutically.

In a fourth aspect, the invention concerns a kit for identifying a woman who has an increased risk for developing venous thrombosis by detecting the presence of genotype AA of the F9 G32023A polymorphism, and/or the presence of the genotype GT6/GT6 of the F9 5:32164:6/4 polymorphism.

In yet another aspect, the invention concerns a method for identifying an agent useful in therapeutically or prophylactically treating venous thrombosis in women, the method comprises detecting the presence of genotype AA of the F9 G32023A polymorphism, and/or the presence of the genotype GT6/GT6 of the F9 5:32164:6/4 polymorphism, and assessing the agent's efficiency in prophylactic and/or therapeutic treatment of venous thrombosis in said women.

In its first aspect, the invention concerns a method for identifying a woman having an increased risk of developing venous thrombosis, the method comprises genotyping the woman with respect to the F9 G32023A polymorphism and/or the F9 5:32164:6/4 polymorphism, wherein detecting the presence of the genotype AA of the F9 G32023A polymorphism and/or the genotype GT6/GT6 of the F9 5:32164:6/4 polymorphism is indicating an increased risk for developing venous thrombosis.

In a non-limiting embodiment of the invention's first aspect, the method for identifying a woman having an increased risk of developing venous thrombosis is a method for identifying a woman's risk of developing venous thrombosis at an age of above 50 years.

In its second aspect, the invention concerns a method for identifying a woman who may benefit from or is in need of prophylactic or therapeutic treatment of venous thrombosis, the method comprises genotyping the woman with respect to the F9 G32023A polymorphism and/or the F9 5:32164:6/4 polymorphism, wherein detecting the presence of the genotype AA of the F9 G32023A polymorphism and/or the genotype GT6/GT6 of the F9 5:32164:6/4 polymorphism is indicating a particular prophylactic or therapeutic treatment of venous thrombosis.

In a non-limiting embodiment of the invention's second aspect, the method for identifying a woman who may benefit from or is in need of prophylactic or therapeutic treatment of venous thrombosis is a method for identifying a woman who has an increased risk of developing venous thrombosis at an age of above 50 years. I.e. the embodiment is particularly a method for identifying a woman who may particularly benefit from or is in need of prophylactic or therapeutic treatment of venous thrombosis at an age of 50 years or older.

In its third aspect, the invention concerns a method for prophylactically or therapeutically treating venous thrombosis in a woman, the method comprises genotyping the woman with respect to the F9 G32023A polymorphism and/or the F9 5:32164:6/4 polymorphism, wherein detecting the presence of the genotype AA of the F9 G32023A polymorphism and/or the genotype GT6/GT6 of the F9 5:32164:6/4 polymorphism is determining the type of prophylactic or therapeutic treatment.

In a particular non-limiting embodiment of the third aspect of the invention, the method for prophylactically or therapeutically treating venous thrombosis in a woman is a method for prophylactically or therapeutically treating venous thrombosis in a woman who is 50 years of age or older.

In an embodiment of the method according to any one of the invention's first, second, or third aspect, the method comprises detection of both the genotypes AA of the F9 G32023A polymorphism and GT6/GT6 of the F9 5:32164:6/4 polymorphism.

In another or additional embodiment of the method according to any one of the invention's first, second, or third aspect, the detection involves a method selected from the group consisting of hybridization-based assays, PCR-based assays, primer extension reactions, sequencing reactions, allele-specific probe hybridization, allele-specific primer extension, allele-specific amplification, sequencing, 5' nuclease digestion, molecular beacon assay, oligonucleotide ligation assay, size analysis, and single-stranded conformation polymorphism.

In another or additional embodiment of the method according to any one of the invention's first, second, or third aspect, the method comprises taking a test sample of the woman.

In another or additional embodiment of the method according to any one of the invention's first, second, or third aspect, genomic DNA of the woman to be risk assessed is used for detecting the genotyping.

According to the fourth aspect, the present invention provides a kit for identifying a woman who has an increased risk of developing venous thrombosis. Said kit is adapted for detecting the presence of the genotype AA of the F9 G32023A polymorphism and/or of the genotype GT6/GT6 of the F9 5:32164:6/4 polymorphism within the genome of a woman.

In an embodiment, the kit comprises at least one "SNP detection reagent" for detecting at least one of the SNPs as specified in any one of SEQ ID NO: 1 to 5, or a complement thereof. As used herein, a "SNP detection reagent" is a reagent which specifically detects a specific target SNP that is selected from the group consisting of the F9 G32023A polymorphism and the F9 5:32164:6/4 polymorphism. Preferably, the SNP detection reagent is specific for a particular nucleotide (allele) of one of said target SNP positions. Most preferably, the SNP detection reagent can differentiate between different alternative nucleotides at a given target SNP position or between different numbers of dinucleotides at a target SNP position, thereby allowing to identify the nucleotide(s) present at the target SNP site to be determined.

In an embodiment, the SNP detection reagent hybridizes to a target SNP-containing nucleic acid molecule by complementary base-pairing in a sequence-specific manner, and discriminates the target variant from other nucleic acid molecules comprising different nucleotide sequences. Preferably, the SNP detection reagent can differentiate between nucleic acids having a particular nucleotide (allele) at a target SNP position from other nucleic acids that have a different nucleotide at the same SNP target position.

In an embodiment of the invention, a SNP detection reagent is a DNA or RNA oligonucleotide probe or primer or PNA oligomer, or a combination thereof, that hybridizes to a segment of a target nucleic acid molecule containing a SNP as specified in any one of SEQ ID NO: 1 to 5. Preferably, the DNA or RNA oligonucleotide probe or primer is an isolated or synthetic DNA or RNA molecule. The SNP detection reagent in form of a polynucleotide may contain modified base analogs, intercalators or minor groove binders. Multiple SNP detection reagents such as probes may be, for example, affixed to a solid support such as an array or beads, or the SNP detection reagents may be supplied in solution (e.g. probe/primer sets for enzymatic reactions such as PCR, RT-PCR, TaqMan assays, or primer-extension reactions) to form a SNP detection kit.

Preferably, the probe or primer is a purified oligonucleotide or PNA oligomer. Such oligonucleotide typically comprises a region of complementary nucleotide sequence that hybridizes under stringent conditions to at least 8, 10, 12, 16, 18, 20, 22, 25, 30, 40, 50, 55, 60, 65, 70, 80, 90, 100, 120 (or any number in-between) or more consecutive nucleotides in a target nucleic acid molecule. The target nucleic acid molecule is a nucleic acid molecule, preferably a DNA molecule, which comprises at least one of the F9 G32023A polymorphism and the GT6/GT6 of the F9 5:32164:6/4 polymorphism. Depending on the particular assay for identifying the genotype of a woman with respect to the F9 G32023A polymorphism and the GT6/GT6 of the F9 5:32164:6/4 polymorphism, the consecutive nucleotides can either include one of the target SNP positions, or be a specific region in close proximity 5' and/or 3' of the SNP position to carry out the desired assay.

Other preferred primer and probe sequences can readily be determined using the transcript sequences, genomic sequences and SNP context sequences disclosed in SEQ ID NO: 6, genomic based context sequences, and complements thereof. It will be apparent to one of skill in the art that such primers and probes are directly useful as reagents for genotyping the SNPs of the present invention, and can be incorporated into any kit/system format.

A primer or probe of the present invention has preferably a length of at least 8 nucleotides. More preferably, the primer or a probe has a length of at least 10 nucleotides. Even more preferably, the primer or a probe has a length of at least 12 nucleotides. Most preferably, the primer or probe has a length of at least 16, 17, 18, 19, 20, 21, 22, 23, 24 or 25 nucleotide. While the maximal length of a probe can be as long as the target sequence to be detected, depending on the type of assay in which it is employed, it is typically less than about 50, 60, 65, or 70 nucleotides in length. In the case of a primer, it is typically less than 30 nucleotides in length. In a specific preferred embodiment of the invention, a primer or a probe is within the length of 18 and 28 nucleotides.

However, in other embodiments of the SNP detection, such as nucleic acid arrays and other embodiments in which probes are affixed to a substrate, the probes can be longer, such as on the order of 30 to 70, 75, 80, 90, 100, or more nucleotides in length.

For analyzing the SNPs or the invention, it is advantageous to use oligonucleotides specific for alternative SNP alleles. Such oligonucleotides that detect single nucleotide variations in target sequences may be referred to by such terms as "allele-specific oligonucleotides", "allele-specific probes", or "allele-specific primers". While the design of each allele-specific primer or probe depends on variables such as the precise composition of the nucleotide sequences flanking a SNP position in a target nucleic acid molecule, and the length of the primer or probe, another factor in the use of primers and probes is the stringency of the condition under which the hybridization between the probe or primer and the target sequence is performed. Higher stringency conditions utilize buffers with lower ionic strength and/or a higher reaction temperature, and tend to require a more perfect match between probe/primer and a target sequence in order to form a stable duplex. If the stringency is too high, however, hybridization may not occur at all. In contrast, lower stringency conditions utilize buffers with higher ionic strength and/or a lower reaction temperature, and permit the formation of stable duplexes with more mismatched bases between a probe/primer and a target sequence. By way of example and not limitation, exemplary conditions for high stringency hybridization conditions using an allele-specific probe are as follows: prehybridization with a solution containing 5X standard saline phosphate EDTA (SSPE), 0.5% NaDodSO₄ (SDS) at 55°C, and incubating probe with target nucleic acid molecules in the same solution at the same temperature, followed by washing with a solution containing 2X SSPE, and 0.1%SDS at 55°C or room temperature.

Moderate stringency hybridization conditions may be used for allele-specific primer extension reactions with a solution containing, e.g., about 50 mM KCl at about 46°C. Alternatively, the reaction may be carried out at an elevated temperature such as 60°C. In another embodiment, a moderately stringent hybridization condition suitable for oligonucleotide ligation assay (OLA) reactions wherein two probes are ligated if they are completely complementary to the target sequence may utilize a solution of about 100 mM KCl at a temperature of 46°C.

In a hybridization-based assay, allele-specific probes can be designed that hybridize to a segment of the target nucleic acid molecule from one individual but do not hybridize to the corresponding segment from another individual due to the presence of different polymorphic forms of the SNP target sites (e.g., alternative SNP alleles/nucleotides) in the respective DNA segments from the two individuals. Hybridization conditions should be sufficiently stringent that there is a significant detectable difference in hybridization intensity between alleles, and preferably an essentially binary response, whereby a probe hybridizes to only one of the alleles or significantly more strongly to one allele. While a probe may be designed to hybridize to a target sequence that contains a SNP site such that the SNP site aligns anywhere along the sequence of the probe, the probe is preferably designed to hybridize to a segment of the target sequence such that the SNP site aligns with a central position of the probe (e.g., a position within the probe that is at least three nucleotides from either end of the probe). This design of probe generally achieves good discrimination in hybridization between different allelic forms.

In another embodiment, a probe or primer may be designed to hybridize to a segment of target nucleic acid molecule such that the SNP aligns with either the 5' most end or the 3' most end of the probe or primer. In a specific preferred embodiment that is particularly suitable for use in a oligonucleotide ligation assay, the 3' most nucleotide of the probe aligns with the SNP position in the target sequence.

Oligonucleotide probes and primers may be prepared by methods well known in the art.

In the case of an array, multiple pairs of probes can be immobilized on the same support for simultaneous analysis of multiple different polymorphisms.

In one type of PCR-based assay, an allele-specific primer hybridizes to a region on a target nucleic acid molecule that overlaps a SNP position and only primes amplification of an allelic form to which the primer exhibits perfect complementarity. Preferably, the primer's 3'-most nucleotide is aligned with and complementary to the SNP position of the target nucleic acid molecule. This primer is used in conjunction with a second primer that hybridizes at a distal site. Amplification proceeds from the two primers, producing a detectable product that indicates which allelic form is present in the test sample. A control is usually performed with a second pair of primers, one of which shows a single base mismatch at the polymorphic site and the other of which exhibits perfect complementarity to a distal site. The single-base mismatch prevents amplification or substantially reduces amplification efficiency, so that either no detectable product is formed or it is formed in lower amounts or at a slower pace. The method generally works most effectively when the mismatch is at the 3'-most position of the oligonucleotide (i.e., the 3'-most position of the oligonucleotide aligns with the target SNP position) because this position is most destabilizing to elongation from the primer. This PCR-based assay can be utilized as part of the TaqMan assay, described below.

In a specific embodiment of the invention, a primer of the invention contains a sequence substantially complementary to a segment of a target SNP-containing nucleic acid molecule except that the primer has a mismatched nucleotide in one of the three nucleotide positions at the 3'-most end of the primer, such that the mismatched nucleotide does not base pair with a particular allele at the SNP site. In a preferred embodiment, the mismatched nucleotide in the primer is the second from the last nucleotide at the 3'-most position of the primer. In a more preferred embodiment, the mismatched nucleotide in the primer is the last nucleotide at the 3'-most position of the primer.

In another embodiment of the invention, a SNP detection reagent of the invention is labeled with a fluorogenic reporter dye that emits a detectable signal. While the preferred reporter dye is a fluorescent dye, any reporter dye that can be attached to a detection reagent such as an oligonucleotide probe or primer is suitable for use in the invention. Such dyes include, but are not limited to, Acridine, AMCA, BODIPY, Cascade Blue, Cy2, Cy3, Cy5, Cy7, Dabcyl, Edans, Eosin, Erythrosin, Fluorescein, 6-Fam, Tet, Joe, Hex, Oregon Green, Rhodamine, Rhodol Green, Tamra, Rox, and Texas Red.

In yet another embodiment of the invention, the detection reagent may be further labeled with a quencher dye such as Tamra, especially when the reagent is used as a self-quenching probe such as a TaqMan or Molecular Beacon probe, or other stemless or linear beacon probe.

The SNP detection reagents of the invention may also contain other labels, including but not limited to, biotin for streptavidin binding, hapten for antibody binding, and oligonucleotide for binding to another complementary oligonucleotide such as pairs of zipcodes.

The present invention also contemplates SNP detection reagents that do not contain (or that are complementary to) a SNP nucleotide identified herein but that are used to assay one or more SNPs disclosed herein. For example, primers that flank, but do not hybridize directly to a target SNP position provided herein are useful in primer extension reactions in which the primers hybridize to a region adjacent to the target SNP position (i.e., within one or more nucleotides from the target SNP site). During the primer extension reaction, a primer is typically not able to extend past a target SNP site if a particular nucleotide (allele) is present at that target SNP site, and the primer extension product can be detected in order to determine which SNP allele is present at the target SNP site. For example, particular ddNTPs are typically used in the primer extension reaction to terminate primer extension once a ddNTP is incorporated into the extension product (a primer extension product which includes a ddNTP at the 3'-most end of the primer extension product, and in which the ddNTP is a nucleotide of a SNP disclosed herein, is a composition that is specifically contemplated by the present invention). Thus, reagents that bind to a nucleic acid molecule in a region adjacent to a SNP site and that are used for assaying the SNP site, even though the bound sequences do not necessarily include the SNP site itself, are also contemplated by the present invention.

A person skilled in the art will recognize that, based on the SNP and associated sequence information disclosed herein, detection reagents can be developed and used to assay any SNP of the present invention individually or in combination, and that a kit may comprise these SNP detection reagents. The terms "kit" and "kits", as used herein in the context of SNP detection reagents, are intended to also refer to such things as combinations of multiple SNP detection reagents, or one or more SNP detection reagents in combination with one or more other types of elements or components (e.g., other types of biochemical reagents, containers, packages such as packaging intended for commercial sale, substrates to which SNP detection reagents are attached, electronic hardware components, etc.). Accordingly, the present invention further provides SNP detection kits, including but not limited to, packaged probe and primer sets (e.g., TaqMan probe/primer sets), arrays/microarrays of nucleic acid molecules, and beads that contain one or more probes, primers, or other detection reagents for detecting one or more SNPs of the present invention. The kits/systems can optionally include various electronic hardware components; for example, arrays ("DNA chips") and microfluidic systems ("lab-on-a-chip" systems) provided by various manufacturers typically comprise hardware components. Other kits/systems (e.g., probe/primer sets) may not include electronic hardware components, but may be comprised of, for example, one or more SNP detection reagents (along with, optionally, other biochemical reagents) packaged in one or more containers.

In some embodiments, a SNP detection kit may further contain one or more other components (e.g., at least one buffer, one or more enzymes such as DNA polymerases or ligases, chain extension nucleotides such as deoxynucleotide triphosphates, and in the case of Sanger-type DNA sequencing reactions, chain terminating nucleotides, positive control sequences, negative control sequences, and the like) necessary to carry out an assay or reaction, such as amplification and/or detection of a SNP-containing nucleic acid molecule. A kit may further contain means for determining the amount of a target nucleic acid, and means for comparing the amount with a standard, and can comprise instructions for using the kit to detect the SNP-containing nucleic acid molecule of interest. In one embodiment of the present invention, kits are provided which contain the necessary reagents to carry out one or more assays to detect one or more SNPs disclosed herein. In a preferred embodiment of the present invention, SNP detection kits/systems are in the form of nucleic acid arrays, or compartmentalized kits, including microfluidic/lab-on-a-chip systems.

The kit for identifying a woman having an increased risk of developing venous thrombosis may contain, for example, one or more probes, or pairs of probes, that hybridize to a nucleic acid molecule at or near each target SNP position. Multiple pairs of allele-specific probes may be included in the kit to simultaneously assay large numbers of SNPs, at least one of which is a SNP of the present invention. Preferably, multiple pairs of allele-specific probes are included in the kit to simultaneously assay both SNPs of the present invention. In some kits, the allele-specific probes are immobilized to a substrate such as an array or bead. The terms "arrays," "microarrays," and "DNA chips" are used herein interchangeably to refer to an array of distinct polynucleotides affixed to a substrate, such as glass, plastic, paper, nylon or other type of membrane, filter, chip, or any other suitable solid support. The polynucleotides can be synthesized directly on the substrate, or synthesized separate from the substrate and then affixed to the substrate.

Any number of probes, such as allele-specific probes, may be implemented in an array, and each probe or pair of probes can hybridize to a different SNP position. In the case of polynucleotide probes, they can be synthesized at designated areas (or synthesized separately and then affixed to designated areas) on a substrate using a light-directed chemical process. Each DNA chip can contain, for example, thousands to millions of individual synthetic polynucleotide probes arranged in a grid-like pattern and miniaturized {e.g., to the size of a dime). Preferably, probes are attached to a solid support in an ordered, addressable array. A microarray can be composed of a large number of unique, single-stranded polynucleotides, usually either synthetic antisense polynucleotides or fragments of cDNAs, fixed to a solid support. Typical polynucleotides are preferably about 6-60 nucleotides in length, more preferably about 15-30 nucleotides in length, and most preferably about 18-25 nucleotides in length. For certain types of microarrays or other detection kits/systems, it may be preferable to use oligonucleotides that are only about 7-20 nucleotides in length. In other types of arrays, such as arrays used in conjunction with chemiluminescent detection technology, preferred probe lengths can be, for example, about 15-80 nucleotides in length, preferably about 50-70 nucleotides in length, more preferably about 55-65 nucleotides in length, and most preferably about 60 nucleotides in length. The microarray or detection kit can contain polynucleotides that cover the known 5' or 3' sequence of a gene/transcript or target SNP site, sequential polynucleotides that cover the full-length sequence of a gene/transcript; or unique polynucleotides selected from particular areas along the length of a target gene/transcript sequence. Polynucleotides used in the microarray or detection kit can be specific to a SNP or SNPs of interest (e.g., specific to a particular SNP allele at a target SNP site, or specific to particular SNP alleles at multiple different SNP sites), or specific to a polymorphic gene/transcript or genes/transcripts of interest.

Hybridization-based assays based on polynucleotide arrays rely on the differences in hybridization stability of the probes to perfectly matched and mismatched target sequence variants. For SNP genotyping, it is generally preferable that stringency conditions used in hybridization assays are high enough such that nucleic acid molecules that differ from one another at as little as a single SNP position can be differentiated (e.g., typical SNP hybridization assays are designed so that hybridization will occur only if one particular nucleotide is present at a SNP position, but will not occur if an alternative nucleotide is present at that SNP position). Such high stringency conditions may be preferable when using, for example, nucleic acid arrays of allele-specific probes for SNP detection. Such high stringency conditions are described in the preceding section, and are well known to those skilled in the art.

Using such arrays, kits or other systems, the present invention provides methods of identifying the SNPs disclosed herein in a test sample. Such methods typically involve incubating a test sample of nucleic acids with an array comprising one or more probes corresponding to at least one SNP position of the present invention, and assaying for binding of a nucleic acid from the test sample with one or more of the probes. Conditions for incubating a SNP detection reagent (or a kit/system that employs one or more such SNP detection reagents) with a test sample vary. Incubation conditions depend on such factors as the format employed in the assay, the detection methods employed, and the type and nature of the detection reagents used in the assay. One skilled in the art will recognize that any one of the commonly available hybridization, amplification and array assay formats can readily be adapted to detect the SNPs disclosed herein.

A SNP detection kit/system of the present invention may include components that are used to prepare nucleic acids from a test sample for the subsequent amplification and/or detection of a SNP-containing nucleic acid molecule. Such sample preparation components can be used to produce nucleic acid extracts (including DNA and/or RNA), proteins or membrane extracts from any bodily fluids (such as blood, serum, plasma, urine, saliva, phlegm, gastric juices, semen, tears, sweat, etc.), skin, hair, cells (especially nucleated cells), biopsies, buccal swabs or tissue specimens. The test samples used in the above-described methods will vary based on such factors as the assay format, nature of the detection method, and the specific tissues, cells or extracts used as the test sample to be assayed. Methods of preparing nucleic acids, proteins, and cell extracts are well known in the art and can be readily adapted to obtain a sample that is compatible with the system utilized. Automated sample preparation systems for extracting nucleic acids from a test sample are commercially available, and examples are Qiagen's BioRobot 9600, Applied Biosystems' PRISM™ 6700 sample preparation system, and Roche Molecular Systems' COBAS AmpliPrep System.

Another form of kit contemplated by the present invention is a compartmentalized kit. A compartmentalized kit includes any kit in which reagents are contained in separate containers. Such containers include, for example, small glass containers, plastic containers, strips of plastic, glass or paper, or arraying material such as silica. Such containers allow one to efficiently transfer reagents from one compartment to another compartment such that the test samples and reagents are not cross-contaminated, or from one container to another vessel not included in the kit, and the agents or solutions of each container can be added in a quantitative fashion from one compartment to another or to another vessel. Such containers may include, for example, one or more containers which will accept the test sample, one or more containers which contain at least one probe or other SNP detection reagent for detecting one or more SNPs of the present invention, one or more containers which contain wash reagents (such as phosphate buffered saline, Tris-buffers, etc.), and one or more containers which contain the reagents used to reveal the presence of the bound probe or other SNP detection reagents. The kit can optionally further comprise compartments and/or reagents for, for example, nucleic acid amplification or other enzymatic reactions such as primer extension reactions, hybridization, ligation, electrophoresis (preferably capillary electrophoresis), mass spectrometry, and/or laser-induced fluorescent detection. The kit may also include instructions for using the kit. Exemplary compartmentalized kits include microfluidic devices known in the art. In such microfluidic devices, the containers may be referred to as, for example, microfluidic "compartments", "chambers", or "channels".

Microfluidic devices, which may also be referred to as "lab-on-a-chip" systems, biomedical micro-electro-mechanical systems (bioMEMs), or multicomponent integrated systems, are exemplary kits/systems of the present invention for analyzing SNPs. Such systems miniaturize and compartmentalize processes such as probe/target hybridization, nucleic acid amplification, and capillary electrophoresis reactions in a single functional device. Such microfluidic devices typically utilize detection reagents in at least one aspect of the system, and such detection reagents may be used to detect one or more SNPs of the present invention. One example of a microfluidic system comprises integration of PCR amplification and capillary electrophoresis in chips. Exemplary microfluidic systems comprise a pattern of microchannels designed onto a glass, silicon, quartz, or plastic wafer included on a microchip. The movements of the samples may be controlled by electric, electroosmotic or hydrostatic forces applied across different areas of the microchip to create functional microscopic valves and pumps with no moving parts. Varying the voltage can be used as a means to control the liquid flow at intersections between the micro-machined channels and to change the liquid flow rate for pumping across different sections of the microchip.

For genotyping SNPs, an exemplary microfluidic system may integrate, for example, nucleic acid amplification, primer extension, capillary electrophoresis, and a detection method such as laser induced fluorescence detection. In a first step of an exemplary process for using such an exemplary system, nucleic acid samples are amplified, preferably by PCR. Then, the amplification products are subjected to automated primer extension reactions using ddNTPs (specific fluorescence for each ddNTP) and the appropriate oligonucleotide primers to carry out primer extension reactions which hybridize just upstream of the targeted SNP. Once the extension at the 3' end is completed, the primers are separated from the unincorporated fluorescent ddNTPs by capillary electrophoresis. The separation medium used in capillary electrophoresis can be, for example, polyacrylamide, polyethyleneglycol or dextran. The incorporated ddNTPs in the single nucleotide primer extension products are identified by laser-induced fluorescence detection. Such an exemplary microchip can be used to process, for example, at least 96 to 384 samples, or more, in parallel.

The nucleic acid molecules of the present invention have a variety of uses, especially in relation to diagnosis and treatment of venous thrombosis in women. For example, the nucleic acid molecules are useful as hybridization probes, such as for genotyping SNPs in messenger RNA, transcript, cDNA, genomic DNA, amplified DNA or other nucleic acid molecules, and for isolating full-length cDNA and genomic clones as well as their orthologs.

A probe can hybridize to a region of a target sequence that encompasses SNP positions of the F9 G32023A polymorphism or of the GT6/GT6 of the F9 5:32164:6/4 polymorphism. More preferably, a probe hybridizes to a SNP-containing target sequence selected from the group consisting of the F9 G32023A polymorphism or the GT6/GT6 of the F9 5:32164:6/4 polymorphism in a sequence-specific manner such that it distinguishes the target sequence from other nucleotide sequences which vary from the target sequence only by which nucleotide is present at the SNP site. Such a probe is particularly useful for detecting the presence of a SNP-containing nucleic acid in a test sample, or for determining which nucleotide (allele) is present at a particular SNP site {i.e., genotyping the SNP site).

A nucleic acid hybridization probe may be used for determining the presence, level, form, and/or distribution of nucleic acid expression. The nucleic acid whose level is determined can be DNA or RNA. Accordingly, probes specific for the SNPs described herein can be used to assess the presence, expression and/or gene copy number in a given cell, tissue, or organism. These uses are relevant for diagnosis of venous thrombosis in women or for assessing a woman's risk of developing venous thrombosis. In vitro techniques for detection of mRNA include, for example, Northern blot hybridizations and in situ hybridizations. In vitro techniques for detecting DNA include Southern blot hybridizations and in situ hybridizations.

Thus, the nucleic acid molecules of the invention can be used as hybridization probes to detect the SNPs disclosed herein, thereby determining whether an individual with the polymorphisms is at risk for venous thrombosis or has developed early stage venous thrombosis. Detection of a SNP associated with a disease phenotype provides a diagnostic tool for an active disease and/or genetic predisposition to the disease.

Furthermore, the nucleic acid molecules of the invention are therefore useful for detecting a factor IX gene which contains a SNP disclosed herein and/or products of such genes, such as expressed mRNA transcript molecules, and are thus useful for detecting gene expression. The nucleic acid molecules can optionally be implemented in, for example, an array or kit format for use in detecting gene expression.

The nucleic acid molecules of the invention are also useful as primers to amplify any given region of a nucleic acid molecule, particularly a region containing a SNP identified herein.

The nucleic acid molecules of the invention are also useful in assays for drug screening to identify compounds that, for example, modulate nucleic acid expression.

The process of determining which specific nucleotide (i.e., allele) is present at each of one or both SNP positions is referred to as SNP genotyping. The present invention provides methods of SNP genotyping, such as for use in screening for venous thrombosis or related pathologies in women, or determining predisposition thereto, or determining responsiveness to a form of treatment, or in genome mapping or SNP association analysis, etc.

Nucleic acid samples can be genotyped to determine which allele(s) is/are present at any given genetic region (e.g., SNP position) of interest by methods well known in the art. The neighboring sequence can be used to design SNP detection reagents such as oligonucleotide probes, which may optionally be implemented in a kit format. Common SNP genotyping methods include, but are not limited to, TaqMan assays, molecular beacon assays, nucleic acid arrays, allele-specific primer extension, allele-specific PCR, arrayed primer extension, homogeneous primer extension assays, primer extension with detection by mass spectrometry, pyrosequencing, multiplex primer extension sorted on genetic arrays, ligation with rolling circle amplification, homogeneous ligation, OLA, multiplex ligation reaction sorted on genetic arrays, restriction-fragment length polymorphism, single base extension-tag assays, and the Invader assay. Such methods may be used in combination with detection mechanisms such as, for example, luminescence or chemiluminescence detection, fluorescence detection, time-resolved fluorescence detection, fluorescence resonance energy transfer, fluorescence polarization, mass spectrometry, and electrical detection.

Various methods for detecting polymorphisms include, but are not limited to, methods in which protection from cleavage agents is used to detect mismatched bases in RNA/RNA or RNA/DNA duplexes, comparison of the electrophoretic mobility of variant and wild type nucleic acid molecules, and assaying the movement of polymorphic or wild-type fragments in polyacrylamide gels containing a gradient of denaturant using denaturing gradient gel electrophoresis (DGGE). Sequence variations at specific locations can also be assessed by nuclease protection assays such as RNase and S1 protection or chemical cleavage methods.

In a preferred embodiment, SNP genotyping is performed using the TaqMan assay, which is also known as the 5' nuclease assay. The TaqMan assay detects the accumulation of a specific amplified product during PCR. The TaqMan assay utilizes an oligonucleotide probe labeled with a fluorescent reporter dye and a quencher dye. The reporter dye is excited by irradiation at an appropriate wavelength, it transfers energy to the quencher dye in the same probe via a process called fluorescence resonance energy transfer (FRET). When attached to the probe, the excited reporter dye does not emit a signal. The proximity of the quencher dye to the reporter dye in the intact probe maintains a reduced fluorescence for the reporter. The reporter dye and quencher dye may be at the 5' most and the 3' most ends, respectively, or vice versa. Alternatively, the reporter dye may be at the 5' or 3' most end while the quencher dye is attached to an internal nucleotide, or vice versa. In yet another embodiment, both the reporter and the quencher may be attached to internal nucleotides at a distance from each other such that fluorescence of the reporter is reduced.

During PCR, the 5' nuclease activity of DNA polymerase cleaves the probe, thereby separating the reporter dye and the quencher dye and resulting in increased fluorescence of the reporter. Accumulation of PCR product is detected directly by monitoring the increase in fluorescence of the reporter dye. The DNA polymerase cleaves the probe between the reporter dye and the quencher dye only if the probe hybridizes to the target SNP-containing template which is amplified during PCR, and the probe is designed to hybridize to the target SNP site only if a particular SNP allele is present.

Preferred TaqMan primer and probe sequences can readily be determined using the SNP and associated nucleic acid sequence information provided herein. A number of computer programs, such as Primer Express (Applied Biosystems, Foster City, CA), can be used to rapidly obtain optimal primer/probe sets. It will be apparent to one of skill in the art that such primers and probes for detecting the SNPs of the present invention are useful in diagnostic assays for VT and related pathologies, and can be readily incorporated into a kit format. The present invention also includes modifications of the Taqman assay well known in the art such as the use of Molecular Beacon probes and other variant formats

Another preferred method for genotyping the SNPs of the present invention is the use of two oligonucleotide probes in an OLA. In this method, one probe hybridizes to a segment of a target nucleic acid with its 3' most end aligned with the SNP site. A second probe hybridizes to an adjacent segment of the target nucleic acid molecule directly 3' to the first probe. The two juxtaposed probes hybridize to the target nucleic acid molecule, and are ligated in the presence of a linking agent such as a ligase if there is perfect complementarity between the 3' most nucleotide of the first probe with the SNP site. If there is a mismatch, ligation would not occur. After the reaction, the ligated probes are separated from the target nucleic acid molecule, and detected as indicators of the presence of a SNP.

In some embodiments, OLA is carried out prior to PCR (or another method of nucleic acid amplification). In other embodiments, PCR (or another method of nucleic acid amplification) is carried out prior to OLA. Another method for SNP genotyping is based on mass spectrometry. Mass spectrometry takes advantage of the unique mass of each of the four nucleotides of DNA. SNPs can be unambiguously genotyped by mass spectrometry by measuring the differences in the mass of nucleic acids having alternative SNP alleles. MALDI-TOF (Matrix Assisted Laser Desorption Ionization - Time of Flight) mass spectrometry technology is preferred for extremely precise determinations of molecular mass, such as SNPs. Numerous approaches to SNP analysis have been developed based on mass spectrometry. Preferred mass spectrometry-based methods of SNP genotyping include primer extension assays, which can also be utilized in combination with other approaches, such as traditional gel-based formats and microarrays.

Typically, the primer extension assay involves designing and annealing a primer to a template PCR amplicon upstream (5') from a target SNP position. A mix of dideoxynucleotide triphosphates (ddNTPs) and/or deoxynucleotide triphosphates (dNTPs) are added to a reaction mixture containing template (e.g., a SNP-containing nucleic acid molecule which has typically been amplified, such as by PCR), primer, and DNA polymerase. Extension of the primer terminates at the first position in the template where a nucleotide complementary to one of the ddNTPs in the mix occurs. The primer can be either immediately adjacent (i.e., the nucleotide at the 3' end of the primer hybridizes to the nucleotide next to the target SNP site) or two or more nucleotides removed from the SNP position. If the primer is several nucleotides removed from the target SNP position, the only limitation is that the template sequence between the 3' end of the primer and the SNP position cannot contain a nucleotide of the same type as the one to be detected, or this will cause premature termination of the extension primer. Alternatively, if all four ddNTPs alone, with no dNTPs, are added to the reaction mixture, the primer will always be extended by only one nucleotide, corresponding to the target SNP position. In this instance, primers are designed to bind one nucleotide upstream from the SNP position (i.e., the nucleotide at the 3' end of the primer hybridizes to the nucleotide that is immediately adjacent to the target SNP site on the 5' side of the target SNP site). Extension by only one nucleotide is preferable, as it minimizes the overall mass of the extended primer, thereby increasing the resolution of mass differences between alternative SNP nucleotides. Furthermore, mass-tagged ddNTPs can be employed in the primer extension reactions in place of unmodified ddNTPs. This increases the mass difference between primers extended with these ddNTPs, thereby providing increased sensitivity and accuracy, and is particularly useful for typing heterozygous base positions. Mass-tagging also alleviates the need for intensive sample-preparation procedures and decreases the necessary resolving power of the mass spectrometer.

The extended primers can then be purified and analyzed by MALDI-TOF mass spectrometry to determine the identity of the nucleotide present at the target SNP position. In one method of analysis, the products from the primer extension reaction are combined with light absorbing crystals that form a matrix. The matrix is then hit with an energy source such as a laser to ionize and desorb the nucleic acid molecules into the gas-phase. The ionized molecules are then ejected into a flight tube and accelerated down the tube towards a detector. The time between the ionization event, such as a laser pulse, and collision of the molecule with the detector is the time of flight of that molecule. The time of flight is precisely correlated with the mass-to-charge ratio (m/z) of the ionized molecule. Ions with smaller m/z travel down the tube faster than ions with larger m/z and therefore the lighter ions reach the detector before the heavier ions. The time-of-flight is then converted into a corresponding, and highly precise, m/z. In this manner, SNPs can be identified based on the slight differences in mass, and the corresponding time of flight differences, inherent in nucleic acid molecules having different nucleotides at a single base position. For further information regarding the use of primer extension assays in conjunction with MALDI-TOF mass spectrometry for SNP genotyping.

SNPs can also be scored by direct DNA sequencing. A variety of automated sequencing procedures can be utilized. The nucleic acid sequences of the present invention enable one of ordinary skill in the art to readily design sequencing primers for such automated sequencing procedures. Commercial instrumentation, such as the Applied Biosystems 377, 3100, 3700, 3730, and 3730x1 DNA Analyzers (Foster City, CA), is commonly used in the art for automated sequencing.

Other methods that can be used to genotype the SNPs of the present invention include single-strand conformational polymorphism (SSCP), and denaturing gradient gel electrophoresis (DGGE). SSCP identifies base differences by alteration in electrophoretic migration of single stranded PCR products. Single-stranded PCR products can be generated by heating or otherwise denaturing double stranded PCR products. Single-stranded nucleic acids may refold or form secondary structures that are partially dependent on the base sequence. The different electrophoretic mobilities of single-stranded amplification products are related to base-sequence differences at SNP positions. DGGE differentiates SNP alleles based on the different sequence-dependent stabilities and melting properties inherent in polymorphic DNA and the corresponding differences in electrophoretic migration patterns in a denaturing gradient gel.

Sequence-specific ribozymes can also be used to score SNPs based on the development or loss of a ribozyme cleavage site. Perfectly matched sequences can be distinguished from mismatched sequences by nuclease cleavage digestion assays or by differences in melting temperature. If the SNP affects a restriction enzyme cleavage site, the SNP can be identified by alterations in restriction enzyme digestion patterns, and the corresponding changes in nucleic acid fragment lengths determined by gel electrophoresis SNP genotyping can include the steps of, for example, collecting a biological sample from a human subject (e.g., sample of tissues, cells, fluids, secretions, etc.), isolating nucleic acids (e.g., genomic DNA, mRNA or both) from the cells of the sample, contacting the nucleic acids with one or more primers which specifically hybridize to a region of the isolated nucleic acid containing a target SNP under conditions such that hybridization and amplification of the target nucleic acid region occurs, and determining the nucleotide present at the SNP position of interest, or, in some assays, detecting the presence or absence of an amplification product (assays can be designed so that hybridization and/or amplification will only occur if a particular SNP allele is present or absent). In some assays, the size of the amplification product is detected and compared to the length of a control sample; for example, deletions and insertions can be detected by a change in size of the amplified product compared to a normal genotype.

SNP genotyping is useful for numerous practical applications, as described below.

Examples of such applications include, but are not limited to, SNP-disease association analysis, disease predisposition screening, disease diagnosis, disease prognosis, disease progression monitoring, determining therapeutic strategies based on an individual's genotype ("pharmacogenomics"), developing therapeutic agents based on SNP genotypes associated with a disease or likelihood of responding to a drug, stratifying a patient population for clinical trial for a treatment regimen, predicting the likelihood that an individual will experience toxic side effects from a therapeutic agent, and human identification applications such as forensics.

According to specific embodiments, detecting may carried out by at least one process selected from the group consisting of allele-specific probe hybridization, allele-specific primer extension, allele-specific amplification, sequencing, 5' nuclease digestion, molecular beacon assay, oligonucleotide ligation assay, size analysis, and single-stranded conformation polymorphism.

In an embodiment of the fourth aspect, the invention concerns a kit for identifying a woman having an increased risk of developing venous thrombosis, said kit comprising at least one SNP detection reagent for detecting at least one of the SNPs specified in any one of SEQ ID Nos. 1 to 5, or a complement thereof.

In an embodiment of the kit, the SNP detection reagent is an isolated or synthetic DNA oligonucleotide probe or primer, or a RNA oligonucleotide or primer or a PNA oligomer or a combination thereof, that hybridizes to a fragment of a target nucleic acid molecule containing one of the SNPs specified in any one of SEQ ID Nos. 1 to 5, or a complement thereof.

In another or additional embodiment of the kit, said SNP detection reagent can differentiate between nucleic acids having a particular nucleotide at a target SNP position.

In another or additional embodiment of the kit, the SNP detection reagent hybridizes under stringent conditions to at least 8, 10, 12, 16, 18, 20, 22, 25, 30, 40, 50, 55, 60, 65, 70, 80, 90, 100, 120 or more consecutive nucleotides in a target nucleic acid molecule comprising at least one of the SNPs specified in any one of SEQ ID Nos. 1 to 5, or a complement thereof.

In another or additional embodiment of the kit, the at least one SNP detection reagent is an oligonucleotide or primer having a length of at least 8 nucleotides, preferably a length of at least 10, 12, 16, 17, 18, 19, 20, 21, 22, 23, 24 or 25 nucleotides.

In another or additional embodiment of the kit, the SNP detection reagent is a compound that is labeled.

In a further aspect, the invention concerns a method for identifying an agent useful in prophylactically or therapeutically treating venous thrombosis in women, the method comprises
- detecting the presence of at least one of the genotypes AA of the F9 G32023A polymorphism and GT6/GT6 of the F9 5:32164:6/4 polymorphism in an experimental model system,
- administering an agent to said experimental model system having said genotype, and
- assessing the efficiency of said agent.

According to a non-limiting embodiment, the model system may be an in-vitro model system, for example a flow chamber model. Such an experimental model system can comprise a flow chamber which may comprise a thrombogenic surface or thrombogenic substrate such as a glass cover slip which is provided with a thrombogenic surface, wherein at least a portion of said thrombogenic surface is utilized as field of interest which is examined for thrombogenesis. The advantage of in-vitro models is the consistency of conditions which may be adjusted as desired. Thus, in-vitro model systems provide the utmost reproducibility.

According to another non-limiting embodiment, said experimental model system may be a cellular model system, preferably a model system comprising mammalian cells, more preferably the cellular model system comprises human cells, most preferably cell of a female humans. Using a cellular model system allows in-vivo experiments under tightly controllable conditions.

According to still another non-limiting embodiment, said experimental model system may be an animal model system. The animal of the animal model system is preferably a mammal, more preferable a female mammal, even more preferably a human, and most preferably a female human. Using an animal model system most closely resembles the clinical situation.

### EXAMPLES

For the initial investigation concerning the identification of genetic variants which are associated with an increased thrombosis risk in elderly persons, a collective of men and women suffering from venous thromboembolism (n=187) as confirmed by duplex sonography and/or computer tomography, and a control collective (n = 200) of healthy blood donors matching age distribution, gender ratio and occurrence of previous and ongoing estrogen therapy of the former collective were built.

For genotyping, leukocytes were extracted from whole blood samples of the persons, and the genomic DNA of the leukocytes was isolated by standard methods. The factor IX gene, in particular the known age-related increase element, was analyzed for polymorphisms by PCR amplification and gene sequencing. DNA fragment having a length of about 408 base pairs (i.e. 408, 410 or 412 bp, depending on polymorphism rs35599944; corresponding to the nucleotide sequence from postion 308 to 715 within SEQ ID NO: 6) were amplified in that primer 1 (3' primer / sense): 5'-CCCTCCATGGTCGTTAAAG-3' (SEQ ID NO: 7) primer 2 (5' primer / antisense): 5'-GTACAATATGTCTGGGTCAATGTC-3' (SEQ ID NO: 8) were derived from the X chromosomal reference sequence (Genebank accession no. NC_000023.9, from position 138472490 to position 138472897) using the program Primer3-web 0.3.0, and used according to the following protocol.

**Table 1: composition of the PCR mix for amplification of a fragment of the F9 gene from human genomic DNA**

| | PCR-Mix (µl) |
|---|---|
| Water | 15.35 |
| 10 X PCR-buffer | 2.19 |
| dATP (10 mM) | 0.44 |
| dCTP (10 mM) | 0.44 |
| dGTP (10 mM | 0.44 |
| dTTP (10 mM) | 0.44 |
| Primer 1 (100 pmol/µl) | 0.23 |
| Primer 2 (100 pmol/µl) | 0.23 |
| MgCl₂ solution | 0.875 |
| Taq DNA polymerase (5 U/µl) | 0.525 |
| Total: | 21.16 |

**Table 2: volume for each reaction in a well of a PCR plate for amplification of a fragment of the F9 gene from human genomic DNA**

| | Volume (µl) |
|---|---|
| PCR mix | 21.5 |
| DNA | 3.5 |
| Total | 25.0 |

**Table 3: PCR program for amplification of a fragment of the F9 gene from human genomic DNA**

| No. of cycles | Duration (s) | Temperature (°C) |
|---|---|---|
| 1 | 600 | 94 |
| 35 | 30 | 94 |
| | 30 | 58 |
| | 30 | 72 |
| 1 | 420 | 72 |
| 1 | ~ | 4 |

Amplified DNA fragments were purified using the QIAquick PCR Purification Kit (QIAGEN, Hilden, Germany) according to the manufacturer's instructions. Purified DNA was dissolved in nuclease-free water at a concentration of between 10 and 20 ng/µl.

Sequencing occurred on a ABI 3130x1 Genetic Analyzer (Applied Biosystems, Weiterstadt, Germany), and the sequencing data were read and analyzed using the programs Finch-TV (Geospize, Seattle, USA) and DNA Baser v2.0 (Plimus, San Jose, USA).

The data were acquired and administered using Microsoft® ACCESS 2000. The statistical analysis was performed using the program SAS® System 9.13 (Statistical Analysis System Deutschland GmbH, Heidelberg, Deutschland).

The Odds-ratios having confidence intervals of 95% were used as estimate value for the relative risk for venous thrombosis. In addition, descriptive statistical methods (means, standard deviation, standard error) were used for comparative illustration of the data. The significance level was set at p > 0.005.

**Table 4: Polymorphisms and occurrence of mutations in an area of the F9 gene**

| gender | Polymorphism | NCBI - SNP-database | Genotype | Number within collective |
|---|---|---|---|---|
| Male | F9 G32023A | rs440051 | A/- | 50(191) |
| Female | F9 G32023A | rs440051 | A/A | 11 (196) |
| | | | G/G | 125(196) |
| | | | G/A | 60(196) |
| Male | F9 GT5:32164:6/4 | rs35599944 | GT4/- | 5 (191) |
| | | | GT5/- | 136 (191) |
| | | | GT6/- | 50 (191) |
| Female | F9 GT5:32164:6/4 | rs35599944 | GT4/GT4 | 0 (196) |
| | | | GT5/GT5 | 116 (196) |
| | | | GT6/GT6 | 11 (196) |
| | | | GT4/GT5 | 9 (196) |
| | | | GT5/GT6 | 55 (196) |
| | | | GT4/GT6 | 5 (196) |

**Table 5: prevalence and relative risk (Odds-ratio) of venous thrombosis in Women associated with variants of two polymorphisms**

| **Polymorphism** | **dbSNP ID** | **Genotype, risk factor** | **Venous thromboembolism % (no. with risk factor/total)** | **Healthy % (no. with risk factor/total)** | **p-value (exact test acc. to Fisher)** | **Odds-ratio (95%CI)** |
|---|---|---|---|---|---|---|
| F9 G32023A | rs440051 | A/A | 10.4 (10/96) | 1.0(1/100) | 0.004 | 11.51 (1.57-500.00) |
| | | G/G | 60.4 (58/96) | 67.0 (67/100) | 0.374 | 0.75 (0.4-1.4) |
| | | G/A | 29.2 (28/96) | 32.0 (32/100) | 0.757 | 0.87 (0.45-1.68) |
| F9 5.32164.6/4 | rs35599944 | GT5/GT5 | 56,3 (54/96) | 62 (62/100) | 0.468 | 0.79 (0.43-1.45) |
| | | GT6/GT6 | 10.4 (10/96) | 1.0(1/100) | 0.004 | 11.51 (1.57-500.00) |
| | | GT4/GT5 | 4.2 (4/96) | 5.0 (5/100) | 1.000 | 0.83 (0.16 -3.97) |
| | | GT5/GT6 | 28.1 /27/96) | 28 (28/100) | 1.000 | 1.00 (0.51 -1.97) |
| | | GT4/GT6 | 1.0 (1/96) | 4.0 (4/100) | 0.370 | 0.25 (0.01-2.63) |

It was found that both, the genotype A/A of the F9 G32023A polymorphism (rs440051) as well as the genotype GT6/GT6 of the F9 5:32164:6/4 polymorphism (rs35599944), which are in fixed coupling imbalance, display p-values of 0.004 in the exact test according to Fisher, and each an odds-ratio of 11.5 (95% confidence interval: 1.57-500.0). This corresponds to an eleven-fold increased risk for women having these genetic variations for thromboembolism.

It was further found that women suffering from venous thrombosis and having the genotype AA of the F9 G32023A polymorphism always had the genotype GT6/GT6 of the F9 5:32164:6/4 polymorphism, and vice versa. Both polymorphisms were previously deposited in the SNP database of the NCBI. However, no data were deposited indicating any clinical relevance of the alleles. Thus, these alleles were identified as predisposing factor for thromboembolism in women.

Upon a more detailed analysis of the data, it was revealed with respect to polymorphism rs440051 that the women at the age of above 50 years which incurred a thromboembolism lead to an Odds ratio of 2.64 (p=0.031, 95% conficence interval was 1.09-6.38) with respect to the genotype AA of said F9 G32023A polymorphism (rs440051) with respect to all controls. The Odds ratio for the women younger than 50 years was found to be 1.0 at p=1.0 (95% conficence interval: 2.38-4.23) whereas the Odds ratio was 1.6 at p= 0.299 (95% conficence interval: 0.74-3.45) for the entirety of women that were included in this analysis.

Hence, the genotype AA of said F9 G32023A polymorphism (rs440051) is a risk factor for women in encountering a thromboembolism, in particular a risk factor indicating an increased risk for women encountering a thromboembolism at an age of 50 years or older.

While the invention has been illustrated and described in detail in the drawings and foregoing description, such illustration and description are to be considered illustrative or exemplary and not restrictive; the invention is not limited to the disclosed embodiments.

Other variations to be disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting scope.

## Claims

1. A method for identifying a woman having an increased risk of developing venous thrombosis, the method comprises genotyping the woman with respect to the F9 G32023A (rs440051) polymorphism and/or the F9 5:32164:6/4 polymorphism (rs35599944), wherein detecting the presence of the genotype AA of the F9 G32023A polymorphism (rs440051) and/or the genotype GT6/GT6 of the F9 5:32164:6/4 polymorphism (rs35599944) is indicating an increased risk for developing venous thrombosis.

2. A method for identifying a woman who may benefit from prophylactic or therapeutic treatment of venous thrombosis, the method comprises genotyping the woman with respect to the F9 G32023A polymorphism (rs440051) and/or the F9 5:32164:6/4 polymorphism (rs35599944), wherein detecting the presence of the genotype AA of the F9 G32023A polymorphism (rs440051) and/or the genotype GT6/GT6 of the F9 5:32164:6/4 polymorphism (rs35599944) is indicating a particular prophylactic or therapeutic treatment of venous thrombosis.

3. A method for prophylactically or therapeutically treating venous thrombosis in a woman, the method comprises genotyping the woman with respect to the F9 G32023A polymorphism (rs440051) and/or the F9 5:32164:6/4 polymorphism (rs35599944), wherein detecting the presence of the genotype AA of the F9 G32023A polymorphism (rs440051) and/or the genotype GT6/GT6 of the F9 5:32164:6/4 polymorphism (rs35599944) is determining the type of prophylactic or therapeutic treatment.

4. The method according to any one of claims 1 to 3, wherein the method comprises detection of both the genotypes AA of the F9 G32023A polymorphism (rs440051) and GT6/GT6 of the F9 5:32164:6/4 polymorphism (rs35599944).

5. The method according to any one of claims 1 to 4, wherein the detection involves a method selected from the group consisting of hybridization-based assays, PCR-based assays, primer extension reactions, sequencing reactions, allele-specific probe hybridization, allele-specific primer extension, allele-specific amplification, sequencing, 5' nuclease digestion, molecular beacon assay, oligonucleotide ligation assay, size analysis, and single-stranded conformation polymorphism.

6. The method according to any one of claims 1 to 5, wherein the method comprises taking a test sample of the woman.

7. The method according to any one of claims 1 to 6, wherein genomic DNA of the woman to be risk assessed is used for detecting the genotyping.

8. A kit for use in identifying a woman having an increased risk of developing venous thrombosis, said kit comprising at least one SNP detection reagent for detecting the presence of the genotype AA of the F9 G32023A polymorphism (rs440051) and/or the genotype GT6/GT6 of the F9 5:32164:6/4 polymorphism (rs35599944).

9. The kit according to claim 8, wherein the SNP detection reagent is an isolated or synthetic DNA oligonucleotide probe or primer, or a RNA oligonucleotide or primer or a PNA oligomer or a combination thereof, that hybridizes to a fragment of a target nucleic acid molecule containing one of the SNPs specified in any one of SEQ ID Nos. 1 to 5, or a complement thereof.

10. The kit according to claim 8 or 9, wherein said SNP detection reagent can differentiate between nucleic acids having a particular nucleotide at a target SNP position.

11. The kit according to any one of claims 8 to 10, wherein the SNP detection reagent hybridizes under stringent conditions to at least 8, 10, 12, 16, 18, 20, 22, 25, 30, 40, 50, 55, 60, 65, 70, 80, 90, 100, 120 or more consecutive nucleotides in a target nucleic acid molecule comprising at least one of the SNPs specified in any one of SEQ ID Nos. 1 to 5, or a complement thereof.

12. The kit according to any one of claims 8 to 11, wherein the at least one SNP detection reagent is an oligonucleotide or primer having a length of at least 8 nucleotides, preferably a length of at least 10, 12, 16, 17, 18, 19, 20, 21, 22, 23, 24 or 25 nucleotides.

13. The kit according to any one of claims 8 to 12, wherein the SNP detection reagent is a compound that is labeled.

14. A method for identifying an agent useful in prophylactically or therapeutically treating venous thrombosis in women, the method comprises
- detecting the presence of at least one of the genotypes AA of the F9 G32023A polymorphism (rs440051) and GT6/GT6 of the F9 5:32164:6/4 polymorphism (rs35599944) in an experimental model system,
- administering an agent to said experimental model system having said genotype, and
- assessing the efficiency of said agent.

15. The method according to claim 14, wherein said experimental model system is selected from the group consisting of in-vitro models, such as a flow-chamber model, cellular model systems, or mammalian model systems, preferably women.
